# EUROPEAN PATENT SPECIFICATION

(11) **EP 4 452 128 B1**
(45) Date of publication and mention of the grant of the patent: **23.07.2025**
(21) Application number: 22834539.3
(22) Date of filing: 08.12.2022
(51) Int. Cl.: A61C 9/00, A61C 17/22, A61C 17/32

(54) **PERSONAL CARE DEVICE, METHOD OF CONTROLLING THE PERSONAL CARE DEVICE AND COMPUTER PROGRAM**
KÖRPERPFLEGEGERÄT, VERFAHREN ZUR STEUERUNG DES KÖRPERPFLEGEGERÄTS UND COMPUTERPROGRAMM
DISPOSITIF DE SOINS PERSONNELS, PROCÉDÉ DE COMMANDE DU DISPOSITIF DE SOINS PERSONNELS ET PROGRAMME INFORMATIQUE

(30) Priority: 23.12.2021 EP 21217431
(43) Date of publication of application: 30.10.2024
(73) Proprietor: Koninklijke Philips N.V., 5656 AG Eindhoven (NL)
(72) Inventor: PALERO, Jonathan Alambra, 5656 AG Eindhoven (NL); VAN DEN DUNGEN, Wilhelmus Andreas Marinus Arnoldus Maria, 5656 AG Eindhoven (NL); JOHNSON, Mark Thomas, 5656AG Eindhoven (NL)
(74) Representative: Philips Intellectual Property & Standards
(86) International application number: PCT/EP2022/084891
(87) International publication number: WO 2023/117441

(56) References cited:
- WO-A1-2021/148764
- US-A1- 2018 177 575

## Description

### FIELD OF THE INVENTION

The present invention relates to the field of personal care devices and methods of controlling them, and in particular to the field of personal care devices that capture digital impressions of one or more features of a user.

### BACKGROUND OF THE INVENTION

It is known to provide personal care devices with the ability or functionality to capture digital impressions (e.g. images) of a feature or part of a user during use. For example, Intra-Oral Scanner (IOS) devices use projected light (e.g. laser or structured light) and an image sensor to capture images of the dentogingival tissues. These images can be processed to create a three-dimensional (3D) model of the scanned surface. Data provided by IOS can therefore be useful for oral care, including the detection of common dental pathologies such as caries, tooth discoloration, misalignment. Also, it may be advantageous to capture and compare repeated IOS images, to enable identification of changes in dentogingival tissues over time for example.

Because personal care devices, such as electric brushing or shaving devices, are used on a regular (e.g. daily) basis, they may provide a suitable vehicle to regularly capture images of a part of a user. Accordingly, there is trend to integrate cameras or imaging sensors into personal care device, such as toothbrushes for example. However, because a main usage characteristic of such a personal care device may be its vibration or cyclical/periodic movement, images acquired by an image capture device integrated with a personal care device are typically distorted and/or blurred by the movement/vibration of the device.

Although image stabilization techniques are known for portable handheld devices (e.g. smart phone, digital photo cameras, etc.), such techniques are only designed and optimized for low frequency (e.g. <10 Hz), erratic (e.g. non-periodic, random, etc.) user-induced motions. Because a personal care device (such as an electric toothbrush) typically moves or vibrates at a much higher periodic frequency (e.g. >20-300 Hz), there remains a need to stabilize the image capture process and/or make the image acquisition process robust to device movements/vibrations generated by a personal care device.

WO 2021/148764 A1 describes a dental treatment appliance which transmits a pulsed light signal towards the oral cavity and captures images of the oral cavity from light returned from the oral cavity.

### SUMMARY OF THE INVENTION

The invention is defined by the claims.

According to examples in accordance with an aspect of the invention, there is provided a personal care device comprising: vibratory means adapted to vibrate the personal care device so that, in use, the personal care device vibrates with a periodic vibration waveform;
an image capture device adapted, in use, to capture images of one or more features of a user; and
a control unit adapted to control an exposure parameter of the image capture device based on a parameter of the periodic vibration waveform.

Proposed concepts thus aim to provide schemes, solutions, concepts, designs, methods and systems pertaining to aiding and/or improving image acquisition from a vibratory personal care device having an image capture device the image of which is influenced by the device vibration. This may either be a camera fixed rigidly to the vibrating device which may then vibrate with the device. Alternatively the camera sensor itself may be situated in a stationary part of a device (e.g. a brush handle) whilst the optical system of the device (like an optical fiber, the imaging lens of the optical system) are subject to the device vibrations. In particular, embodiments of the invention propose controlling an exposure parameter of the image capture device based on a parameter of the periodic vibration waveform. Through control of an exposure parameter of the image capture device, improved images (e.g. images exhibiting less blur and/or distortion) may be obtained. In this way, improved images may be obtained from a vibratory personal care device.

In particular, it is proposed that, to reduce or minimize distortions in images acquired from vibrating personal care device (in use), exposure settings of the image capture device (which also vibrates as part of the personal care device) may be adapted based upon a parameter of the periodic vibration waveform.

In other words, embodiments propose to adapt image capture to one or more characteristics of the vibration of the personal care device, so as to provide images of improved quality. Embodiments may therefore facilitate stable and high-quality image acquisition during tooth brushing. Such embodiments may be particularly relevant to teledentistry propositions, for example, by enabling improved imaging of a user's tooth, gum, tongue, etc. For instance, images obtained by proposed embodiments may aid dental care treatments and/or decision making. Accordingly, embodiments may be used in relation to dental treatment selection so as support a dental care professional when selecting treatment for a subject.

Further, embodiments may facilitate image-based diagnostics, superior location sensing, therapy planning (e.g. orthodontics, aligners) and/or dental treatment monitoring.

By being integrated into the normal brushing regimen of a user (instead of using separate devices such as smart phones, dental endo-scopes or handheld intraoral scanners, embodiments may support improved dental care. Improved image-based dental care may therefore be provided by proposed concepts.

It has been realized that by controlling one or more exposure parameters of the image capture device based on the vibration cycle of the personal care device, higher quality images (in terms of reduced blur and/or distortion) may be obtained, thus aiding oral/dental care decision making.

Embodiments may therefore provide the advantage that decision making in selecting an oral care treatment can be improved through the use of images captured by a virbatory personal care device. For example, embodiments may enable a larger number of oral care treatment options to be available (e.g. through the provision of more and/or improved information about a subject's oral health).

According to the invention, the exposure parameter comprises an image capture exposure duration of the image capture device. The control unit is then adapted to set the image capture exposure duration to be less than or equal to a target exposure duration value. The target exposure duration value may, for example, be less than or equal to the time taken for image capture by the image capture device (i.e. exposure shift) to move across a predetermined number of pixels, preferably less than a single pixel.

The target exposure duration value is determined based on a parameter of the periodic vibration waveform and a resolution parameter of the image capture device. For instance, the at least one resolution parameter may comprise one or more of: sensor pixel width; sensor pixel length; sensor spacing; and sensor density. In this way, the exposure duration may be set to an optimum value relation to the imaging sensor exposure shift during specific moments of motion, so as to prevent image exposure across multiple sensor elements/pixels. That is, an exposure duration of the image capture device may be set to be fast enough relative to the camera vibration to reduce or minimize image distortion or blur. Also, the total exposure duration may be shared (i.e. divided) across all rows of the camera sensor, so that light is incident on the sensor for short pulsed period per row.

By way of example, the control unit may be adapted to determine the target exposure duration value based on the parameter of the periodic vibration waveform and at least one resolution parameter of the image capture device. The at least one resolution parameter may, for example, comprise one or more of: sensor pixel width; sensor pixel length; sensor spacing; and sensor density. Reduction of the exposure duration may prevent pixel shift during exposure, e.g. by ensuring that exposure of an image camera pixel is from the same position and thus will not blur with the next pixel position

Embodiments may therefore be particularly useful in combination with free running image capture devices.

The image capture device may also comprise an illumination device configured, in use, to illuminate a part of the user cavity. The control unit may then also be adapted to control the image capture device to synchronize a timing of illumination with the vibration cycle. This may help to ensure adequate lighting conditions for reducing the required length/duration of exposure for image capture. It may also help to reduce power consumption by restricting illumination to only occur at certain times. That is, activation of the illumination device for unnecessary time periods may be avoided.

In some embodiments, the control unit may be adapted to control an image capture frame rate of the image capture device based on the vibration frequency of the personal care device.

Also, in some embodiments, the control unit may be configured to control the vibratory means to adjust the vibration frequency of the personal care device based on an image capture frame rate of the camera.

Embodiments may be employed in conjunction with different types of image capture devices, including, for example, global shutter cameras or free-running rolling shutter cameras that are out of synchronization with vibration of the personal care device. For example, by setting the exposure in relation to the frame capture rate of the camera, image frames that are sharp and clear may be captured.

In yet further exemplary embodiments, the control unit may be adapted to distribute the exposure parameter across a frame of the image capture device, preferably on a line-by-line basis or an area-by-area basis. The control unit may also be further adapted to vary the exposure parameter across the frame of the image capture device.

The personal care device may comprise a toothbrush, and may preferably comprises an electric toothbrush that is adapted to vibrate in use. In other embodiments, the personal care device may comprise a mouthpiece, shaver or a skin cleansing device. One or more proposed concept(s) may therefore be employed in a range of different personal care devices. Embodiments may therefore have wide application in the field of personal care devices (and image capture and/or processing concepts for images captured by vibratory personal care devices).

According to another aspect of the invention, there is provided a method of controlling a personal care device, the personal care device comprising: vibratory means adapted to vibrate the personal care device so that, in use, the personal care device vibrates with a periodic vibration waveform; and an image capture device adapted, in use, to capture images of one or more features of a user, wherein the method comprises: controlling an exposure parameter of the image capture device based on a parameter of the periodic vibration waveform.

The image capture device may comprise an illumination device configured, in use, to illuminate a part of the user cavity. Controlling an exposure parameter of the image capture device may then comprise controlling the image capture device to synchronise a timing of illumination with the vibration waveform.

According to yet another aspect of the invention, there is provided a computer program comprising computer program code means which is adapted, when said computer program is run on a computer, to implement a method according to proposed embodiment.

Thus, there may also be provided a computer system comprising: a computer program product according to proposed embodiment; and one or more processors adapted to perform a method according to a proposed concept by execution of the computer-readable program code of said computer program product.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a simplified schematic block diagram of an electric toothbrush 10 according to a proposed embodiment;
Figure 2 is a graph showing an exemplary variation in angle θ (of displacement) and angular velocity of the brush head of Figure 1 over time as the brush head vibrates, wherein the variation in displacement angle θ of the brush head is depicted by the solid line, and wherein the variation in angular velocity v of the brush head 14 is depicted by the dashed line;
Figure 3 is a simplified schematic block diagram of a mouthpiece according to a proposed embodiment;
Figure 4 illustrates results of a simulation of captured image frames using a vibrating camera having image capture frame rate of 30 frames per second for varying camera exposure times, vibration frequencies and vibration periods; and
Figure 5 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for aiding and/or improving image acquisition from a vibratory personal care device having an image capture device where the camera image vibrates together with the device. In particular, embodiments may provide a system, device and/or method which controls an exposure parameter of the image capture device based on a parameter of the vibration of vibratory personal care device. Through such control of an exposure parameter of the image capture device, improved images (e.g. images exhibiting less blur and/or distortion) may be obtained.

In particular, it is proposed that, to reduce or minimize distortions in images acquired from a vibrating personal care device, one or more exposure settings of the image capture device may be adapted according to the vibration cycle of the vibrating personal care device (in use). In other words, embodiments propose to adapt image acquisition to the vibration of the personal care device, so as to provide images of improved quality.

Embodiments may therefore facilitate stable and high-quality image acquisition during tooth brushing. Such embodiments may be particularly relevant to teledentistry propositions, for example, by enabling improved imaging of a user's tooth, gum, tongue, etc. For instance, images obtained by proposed embodiments may aid dental care treatments and/or decision-making. Accordingly, embodiments may be used in relation to dental treatment selection so as support a dental care professional when selecting treatment for a subject.

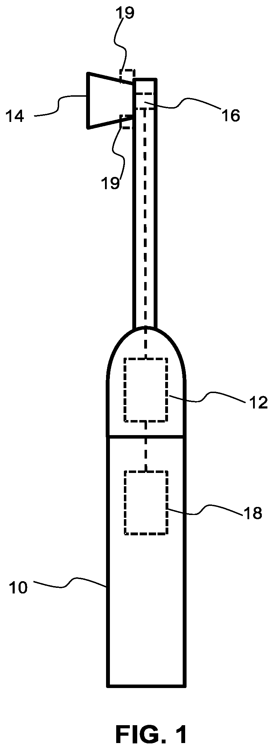

Referring to Figure 1, there is shown a simplified schematic block diagram of an electric toothbrush 10 according to a proposed embodiment. The electric toothbrush 10 comprises vibratory means 12 (specifically, a motor) that is adapted, in use, to vibrate a brush head 14 of the electric toothbrush 10 (with a frequency of about 200Hz).

The electric toothbrush 10 also comprises an image capture device 16 (e.g. digital camera) that is adapted, in use, to capture images of one or more oral features of a user. The electric toothbrush 10 is also provided with a control unit 18 that is adapted to control an exposure parameter of the image capture device 16.

More specifically, the motor 12 is configured to cause the brush head 14 to repeatedly rotates clockwise then anti-clockwise by around 10°-25° from a rest position in a periodic manner. In this way, the brush head 14 (and the image capture device 16) vibrates with a periodic vibration waveform. That is, the image capture device 16 vibrates together with the brush head 14.

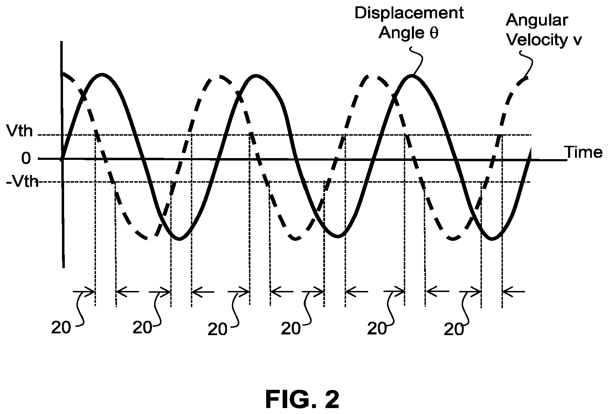

By way of further illustration, Figure 2 depicts a graph showing an exemplary variation in angle θ (of displacement) and angular velocity of the brush head 14 over time as the brush head 14 vibrates. The variation in displacement angle θ of the brush head 14 is depicted by the solid line, whereas the variation in angular velocity v of the brush head 14 is depicted by the dashed line.

It is seen from the variations depicted in Figure 2 that 'low speed' periods 20, i.e. time periods wherein the magnitude of angular velocity v of the brush head 14 is below a threshold value Vth, can be identified. More specifically, it is seen that, when the magnitude of the displacement angle θ reaches a maximum value, such that the gradient of displacement angle θ variation changes polarity (i.e. when the solid line changes direction), the angular velocity v of the brush head 14 is near-zero. It is proposed that these low speed periods 20 provide the most appropriate opportunities for image capture using an image capture device that may have limited speed and exposure tuning capabilities. Accordingly, embodiments are based on the concept(s) that one or more exposure settings of the image capture device 16 may be adapted according to the vibration cycle of the brush 14. That is, embodiments propose to adapt image acquisition to the vibration of the personal care device, so as to provide images of improved quality.

Turning back to the specific embodiment of Figure 1, the control unit 18 is adapted to control an exposure parameter of the image capture device 16 based on a parameter of the periodic vibration waveform.

The exposure parameter comprises an exposure duration (i.e. length of exposure) of the image capture device, and the control unit 18 is adapted to set the image capture exposure duration to be equivalent to the time required for the image to move less than one pixel shift on the camera sensor as a result of the vibration. It may be preferable, however, to limit the exposure duration to shorter periods (i.e. lower duration values), so as to allow image capture for higher speed motion.

Moreover, for the case of a rolling shutter camera for example, it may be even more preferable to limit the exposure duration further on a line-by-line basis to be equivalent to less than one pixel shift on the image capture device - that is, the shift of the image during motion that is within the pixel resolution. In this way, the total exposure duration may be shared (i.e. divided) across all rows of the camera sensor, so that light is incident on the sensor for short pulsed exposure period per row, with the cumulative total duration of all of the pulses adding up to the total exposure duration. This may be implemented by exposing each row of the sensor to incident light for a fraction of the exposure duration or by pulsing an illumination source for fraction of the exposure duration with the pulsing synchronized with the row exposure time of each row of the rolling shutter camera sensor.

As an example, with the distance to the object fixed, and motion fixed, the pixel shift speed may be calculated. For instance, if the image capture device has a sensor with 1024*768 pixels, the image area captured being 10 mm wide, and motion image shift being 2 mm left and 2 mm right with a vibration period of 2 ms. Movement will thus be 4mm in ½ period (1ms), and 4mm is covered by 4/10*1024 = 410 pixels. A single pixel image movement takes 1ms/410 = 2.4µs. Allowing for ½ a pixel shift during exposure would result in an exposure time of 1.2µS. Thus, a total exposure time is the number of rows in the image times the line exposure time i.e.768 x 1.2µs = 0.92msec. In such a manner, the short pulsed exposure periods are distributed evenly across the entire frame period and exposure is optimized for and during the low speed motion periods.

Here, reference to pixel may encompass a single sensing element of an image sensor or a sensing element (single sensor) of an image sensor array. That is, a pixel may be thought of as being a single element of a sensed image, wherein the sensed image is formed from a plurality of sensed elements (i.e. pixels).

In other cases like high framerates and special camera's where per line or per pixel the exposure can be adjusted, an optimum per pixel, line or frame could be set during the image acquisition and in relation to the ideal moments of capture where for example the speed of motion is low.

Furthermore, in an example, the exposure parameter comprises a phase of exposure timing (i.e. the phase of the vibration at which an exposure is commenced/started), and the control unit 18 is adapted to control the image capture device 16 to synchronize the exposure timing with the phase of the vibration cycle. Specifically, the control unit 18 is adapted to control the image capture device 16 to synchronize the exposure timing with the low speed periods 20 and high speed periods of the vibration cycle. In this way, the image capture device 16 is controlled so that image capture exposure is synchronized with the different periods, thus reducing an amount of movement that may cause blurring and/or distortion in a captured image, as described below.

In a further development, the exposure could even be adjusted as a function of the phase of the vibration i.e. whether we are in the low speed or high speed motion phase of the vibration. In this manner, there may also be advantages in distributing the exposure periods in an uneven manner across the entire frame period or even over a succession of frame periods. Therefore, in a further development of a beneficial uneven distribution of such exposure periods, it is noted that there are portions of the vibration, for example where the motion of the vibration is low or zero, where high quality images can be realized for relatively longer exposures, and portions of the vibration, for example where the motion of the vibration is high, where high quality images can only be realized for shorter exposures. For example, it could be advantageous to collect good quality image data during the low and high motion portions (20) of Figure 2. In a non-limiting example this could be achieved by operating the camera with a frame rate which is eight times higher than the vibration frequency. In such an approach, it would be possible to concentrate the longer pulsed exposure period per row into the low motion portions (20) of Figure 2. Similarly, a shorter exposure period per row would be applied to the areas adjacent to the low motion portions (20) of figure 2. Finally, an even shorter exposure period per row would be applied to the high motion portions (20) of figure 2. By concentrating the longer and shorter exposure periods in these portions of the vibration an uneven distribution of exposure across the vibration period is achieved.

To aid reduction in the exposure duration that is required to capture an adequately exposed image, the image capture device 16 of the embodiment of Figure 1 comprises illumination devices 19 (e.g. LEDs) that are configured, in use, to illuminate a part of the user's oral cavity. That is, where the sensitivity (e.g. ISO) and/or the lens aperture of the image capture device 16 may be fixed and/or limited, a reduction in the required length of exposure to capture an adequate amount of light may be obtained through illumination of the user's oral cavity by the illumination devices 19. This may, for example, be implemented by pulsing an illumination source for fraction of the exposure duration with the pulsing synchronized with the row exposure time of each row of the rolling shutter camera sensor. In other embodiments, however, the illumination devices 19 may be controlled to provide continuous illumination, e.g. illumination may be provided by the illumination devices 19 for the duration of the vibration cycle.

Although the embodiment of Figure 1 has been described as employing a brush head 14 that repeatedly rotates clockwise then anti-clockwise by around 10°-25° from a rest position in a periodic manner, it will be appreciated that other embodiments may employ a brush head that vibrates in a different manner. For instance, alternative embodiments may comprise a brush head that rotates or that shakes or sweeps (left and right, or up and down) repeatedly, i.e. repeatedly displaces (laterally or vertically) in opposite directions from a rest position. Such embodiments will still exhibit a cyclical vibration pattern having variations in displacement and velocity over time that form periodic waveforms as depicted in Figure 2, and thus have repeating 'low speed' periods that are identifiable (e.g. according to the drive parameters and/or control of the vibratory means).

That is, although the proposed concept(s) have been described above with reference to rotating / angular motion of the vibrating part of the personal care device, the proposed concept(s) may be employed in other vibratory personal care devices exhibiting repetitive vibratory motion. Also, in case of multi-dimensional motion, periods of low/zero speed may be identified with respect to multiple dimensions and used to control one or more exposure parameters of the image capture device.

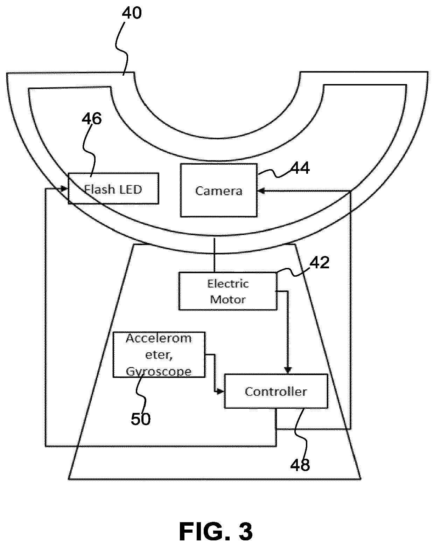

Referring to Figure 3, there is shown a simplified schematic block diagram of a mouthpiece 40 according to a proposed embodiment. The mouthpiece 40 is adapted to be inserted into a user's mouth and vibrate (in use) for oral cleaning purposes.

The mouthpiece 40 comprises vibratory means 42 (specifically, an electric motor) that is adapted, in use, to cause the mouthpiece to vibrate with periodic vibration waveform (having a frequency in the range of 100Hz-500Hz).

The mouthpiece 40 also comprises an image capture device 44 positioned in the tray of the mouthpiece and adapted, in use, to capture images of one or more oral features of the user. A flash LED 46 is also provided in the tray of the mouthpiece for illuminating the oral cavity of the user, in use. The image capture device 44 and LED 46 are configured to be controlled by a control unit (i.e. controller) 48 of the mouthpiece.

In particular, the control unit 48 is configured to control an exposure parameter of the camera 44 and activation the flash LED 46 based on a parameter of the periodic vibration waveform of the mouthpiece 40. For determining a parameter of the periodic vibration waveform of the mouthpiece, the mouthpiece 40 comprises a sensor arrangement 50 that is adapted to detect properties of the movement/vibration of the mouthpiece, although this is optional and the vibratory properties may be derived from the motor driver. Specifically, in this example, the sensor arrangement 50 comprises an accelerometer and gyroscope for detecting variations in displacement and velocity of the mouthpiece 40. Information about the detected variations in displacement and velocity of the mouthpiece 40 is provided to the control unit 48 and, based on this information, the control unit 48 determines parameters of the periodic vibration waveform of the mouthpiece 40 (such as period, frequency, amplitude, zero-crossing timings, etc.).

It is noted that, in this embodiment, the image capture device 44 comprises a rolling shutter camera configured to operate at an image capture frame rate. With respect to such a rolling shutter camera, trade-offs in operating characteristics may be required. For example, rolling shutter cameras may be more cost effective (e.g. cheaper) than a global shutter camera but may take longer for a full image capture depending on the acquisition settings and/or capabilities. Accordingly, the control unit 48 is thus further is adapted to control an exposure parameter taking accounting of the frame rate of the rolling shutter camera.

That is, for image acquisition using a rolling shutter camera 44, it is proposed to adapt the exposure duration to be fast relative to the camera vibration, e.g. effective exposure time (i.e. image data acquisition time from the rolling shutter camera 44) is adapted to be equivalent to the time required for the image to move less then one pixel shift on the camera sensor as a result of the vibration. In this way, a conventional slow/cheap rolling shutter camera may be employed, because the exposure can be tuned to the low speed moments (thus alleviating a need for fast camera capture to match the vibration speed).

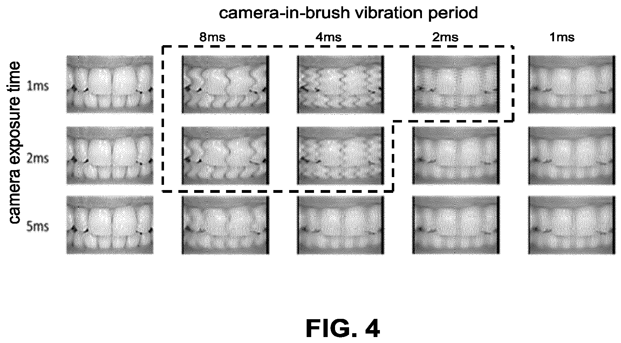

Referring to Figure 4, results of a simulation of captured image frames using a vibrating camera at 30 frames per second are illustrated for varying camera exposure times, vibration frequencies and vibration periods. The camera exposure time here represents the total camera exposure time during the captured frame. As described above, in preferred embodiments the total camera exposure time is distributed across the individual captured lines of the image, whereby line exposure times in the order of microseconds are realized. The exposure time may be distributed evenly across the frame, or may also be distributed according to the phase of the vibratory motion i.e. differently for low speed and high speed areas, as described above.

In dashed box are captured images that are restorable. This is far below the speeds as being used by for example high speed camera's capable of high frame rates and short exposure times, as indicated in the capabilities of commonly used affordable rolling shutter cameras, due to the fact the line capture speed is very high. When the exposure time is optimized in relation to this brushing motion and camera frame rate, it is observed that sharp and clear areas are present in the captured image frames. Further, with basic algorithms (like edge detection) features can be found and used in a full image reconstruction.

Thus, it will be appreciated that the rolling shutter camera can be free running and out of synchronization with vibration of the personal care device (and thus the image data capture frequency). A full image may then be reconstructed from image data acquired from multiple difference frames of the rolling shutter camera.

By way of example, embodiments may employ a concept of processing captured images from the rolling shutter camera. For such a concept, the control unit may comprise a processor arrangement and an image processor. The process arrangement may process a captured image from the rolling shutter camera with an image processing algorithm to identify a target region of the captured image comprising image data having a quality characteristic value (e.g. sharpness, edge quality, distortion, brightness, contrast etc.) that meets a first predetermined requirement. The image processor may then extract image data from the identified region of the captured image and generate a reconstructed image comprising the extracted image data. In this way, high quality regions of captured images may be identified and then identified high-quality regions of a plurality of different images may be employed to build up (i.e. construct) a single, high-quality image.

Furthermore, in some embodiments, the control unit may have an interface configured to obtain, as reference image, an image captured by the rolling shutter camera. The image processor may then: process a segment of the captured image with the image processing algorithm to determine the quality characteristic value of the image data of the segment; compare the quality characteristic value of the image data of the segment with a quality characteristic value of image data of a corresponding region of the reference image; and, based on the comparison result, identify the segment as a region of the captured image comprising image data having quality characteristic value meeting the first predetermined requirement. Image segments comprising image data of higher quality than the reference image may thus be identified and used to construct an improved/optimal image from multiple different captured images.

From the above description of various concepts and embodiments, it will be appreciated that there is proposed a method of controlling a personal care device, the personal care device comprising: vibratory means adapted to vibrate the personal care device so that, in use, the personal care device vibrates with a periodic vibration waveform; and an image capture device adapted, in use, to capture images of one or more oral features of a user. The proposed method comprises: controlling an exposure parameter of the image capture device based on a parameter of the periodic vibration waveform. Such a method may be employed in a processing system or computer, and such a system/computer may be integrated with a vibratory personal care device.

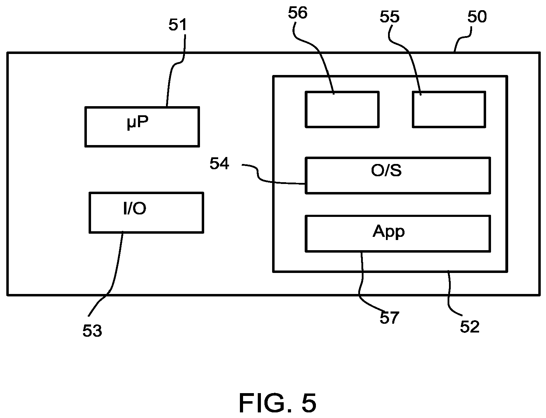

Figure 5 illustrates an example of a computer 50 within which one or more parts of an embodiment may be employed. Various operations discussed above may utilize the capabilities of the computer 500. For example, one or more parts of a system for providing a subject-specific user interface may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 500 includes, but is not limited to, PCs, workstations, laptops, PDAs, palm devices, smartphones, smartwatches, servers, storages, and the like. Generally, in terms of hardware architecture, the computer 500 may include one or more processors 51, memory 52, and one or more I/O devices 53 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 51 is a hardware device for executing software that can be stored in the memory 52. The processor 51 can be virtually any custom made or commercially available processor, a central processing unit (CPU), a digital signal processor (DSP), or an auxiliary processor among several processors associated with the computer 50, and the processor 51 may be a semiconductor based microprocessor (in the form of a microchip) or a microprocessor.

The memory 52 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 52 may incorporate electronic, magnetic, optical, and/or other types of storage media. Note that the memory 52 can have a distributed architecture, where various components are situated remote from one another, but can be accessed by the processor 51.

The software in the memory 52 may include one or more separate programs, each of which comprises an ordered listing of executable instructions for implementing logical functions. The software in the memory 52 includes a suitable operating system (O/S) 54, compiler 56, source code 55, and one or more applications 57 in accordance with exemplary embodiments. As illustrated, the application 57 comprises numerous functional components for implementing the features and operations of the exemplary embodiments. The application 57 of the computer 50 may represent various applications, computational units, logic, functional units, processes, operations, virtual entities, and/or modules in accordance with exemplary embodiments, but the application 57 is not meant to be a limitation.

The operating system 54 controls the execution of other computer programs, and provides scheduling, input-output control, file and data management, memory management, and communication control and related services. It is contemplated by the inventors that the application 57 for implementing exemplary embodiments may be applicable on all commercially available operating systems.

Application 57 may be a source program, executable program (object code), script, or any other entity comprising a set of instructions to be performed. When a source program, then the program is usually translated via a compiler (such as the compiler 56), assembler, interpreter, or the like, which may or may not be included within the memory 52, so as to operate properly in connection with the O/S 54. Furthermore, the application 57 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 53 may include input devices such as, for example but not limited to, a mouse, keyboard, scanner, microphone, camera, etc. Furthermore, the I/O devices 53 may also include output devices, for example but not limited to a printer, display, etc. Finally, the I/O devices 53 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 53 also include components for communicating over various networks, such as the Internet or intranet.

If the computer 50 is a PC, workstation, intelligent device or the like, the software in the memory 52 may further include a basic input output system (BIOS) (omitted for simplicity). The BIOS is a set of essential software routines that initialize and test hardware at startup, start the O/S 54, and support the transfer of data among the hardware devices. The BIOS is stored in some type of read-only-memory, such as ROM, PROM, EPROM, EEPROM or the like, so that the BIOS can be executed when the computer 800 is activated.

When the computer 50 is in operation, the processor 51 is configured to execute software stored within the memory 52, to communicate data to and from the memory 52, and to generally control operations of the computer 50 pursuant to the software. The application 57 and the O/S 54 are read, in whole or in part, by the processor 51, perhaps buffered within the processor 51, and then executed.

When the application 57 is implemented in software it should be noted that the application 57 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 57 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed image capture and/or processing methods, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 1 and 3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A personal care device comprising:
vibratory means (12) adapted to vibrate the personal care device so that, in use, the personal care device vibrates with a periodic vibration waveform;
an image capture device (16) adapted, in use, to capture images of one or more features of a user; and
a control unit (18) adapted to control an exposure parameter of the image capture device based on a parameter of the periodic vibration waveform;
wherein the exposure parameter comprises an image capture exposure duration of the image capture device, and wherein the control unit is adapted to set the image capture exposure duration to be less than or equal to a target exposure duration value; and
wherein the control unit (18) is adapted to determine the target exposure duration value based on the parameter of the periodic vibration waveform and at least one resolution parameter of the image capture device, preferably wherein the at least one resolution parameter comprises one or more of: sensor pixel width; sensor pixel length; sensor spacing; and sensor density.

2. The device of claim 1, wherein the target exposure duration value is less than or equal to the time taken for image capture by the image capture device to move across a predetermined number of pixels, preferably wherein the predetermined number of pixels is one pixel.

3. The device of any of the preceding claims, wherein the personal care device comprises an oral care device, and wherein the image capture device (16) is adapted, in use, to capture images of one or more oral features of a user.

4. The device of any of the preceding claims, wherein the image capture device comprises an illumination device (19) configured, in use, to illuminate a part of the user, and preferably wherein the control unit is adapted to control the image capture device to synchronize a timing of illumination with the vibration waveform.

5. The device of any of the preceding claims, wherein the control unit (18) is adapted to control an image capture frame rate of the image capture device further based on the vibration frequency of the personal care device.

6. The device of any of the preceding claims, wherein the control unit (18) is further configured to control the vibratory means to adjust the vibration frequency of the personal care device based on an image capture frame rate of the camera.

7. The device of any of the preceding claims, wherein the control unit (18) is adapted to distribute the exposure parameter across a frame of the image capture device, preferably on a line-by-line basis.

8. The device of claim 7, wherein the control unit (18) is further adapted to vary the exposure parameter across the frame of the image capture device.

9. The device of any of the preceding claims, wherein the personal care device comprises a toothbrush.

10. The device of any of the preceding claims, wherein the personal care device comprises a mouthpiece.

11. A method of controlling a personal care device, the personal care device comprising: vibratory means adapted to vibrate the personal care device so that, in use, the personal care device vibrates with a periodic vibration waveform; and an image capture device adapted, in use, to capture images of one or more features of a user, wherein the method comprises:
controlling an exposure parameter of the image capture device based on a parameter of the periodic vibration waveform;
determining a target exposure duration value based on the parameter of the periodic vibration waveform and at least one resolution parameter of the image capture device;
setting the image capture exposure duration to the target exposure duration value,
and preferably wherein the at least one resolution parameter comprises one or more of: sensor pixel width; sensor pixel length; sensor spacing; and sensor density.

12. A computer program comprising computer program code means which is adapted, when said computer program is run on the personal care device of claim 1, to implement the method of claim 11.

## Patentansprüche

1. Körperpflegevorrichtung, umfassend:
Vibrationsmittel (12), die dazu eingerichtet sind, die Körperpflegevorrichtung in Vibrationen zu versetzen, sodass die Körperpflegevorrichtung in Verwendung mit einer periodischen Vibrationswellenform vibriert;
eine Bildaufnahmevorrichtung (16), die dazu eingerichtet ist, in Verwendung Bilder von einem oder mehreren Merkmalen eines Benutzers aufzunehmen; und
eine Steuereinheit (18), die dazu geeignet ist, einen Belichtungsparameter der Bildaufnahmevorrichtung basierend auf einem Parameter der periodischen Vibrationswellenform zu steuern;
wobei der Belichtungsparameter eine Belichtungsdauer für die Bildaufnahme der Bildaufnahmevorrichtung umfasst und wobei die Steuereinheit dazu eingerichtet ist, dass sie die Belichtungsdauer für die Bildaufnahme so festlegt, dass sie kleiner als oder gleich einem Zielbelichtungsdauerwert ist; und
wobei die Steuereinheit (18) eingerichtet ist, den Zielbelichtungsdauerwert auf der Grundlage des Parameters der periodischen Vibrationswellenform und mindestens eines Auflösungsparameters der Bildaufnahmevorrichtung zu bestimmen, wobei der mindestens eine Auflösungsparameter vorzugsweise eines oder mehrere der folgenden Elemente umfasst: Sensorpixelbreite; Sensorpixellänge; Sensorabstand; und Sensordichte.

2. Die Vorrichtung nach Anspruch 1, wobei der Zielbelichtungsdauerwert kleiner oder gleich der Zeit ist, die für die Bildaufnahme durch die Bildaufnahmevorrichtung benötigt wird, um sich über eine vorbestimmte Anzahl von Pixeln zu bewegen, wobei die vorbestimmte Anzahl von Pixeln vorzugsweise ein Pixel ist.

3. Vorrichtung nach einem der vorstehenden Ansprüche, wobei die Körperpflegevorrichtung eine Mundpflegevorrichtung umfasst, und wobei die Bildaufnahmevorrichtung (16) dazu eingerichtet ist, in Verwendung Bilder von einem oder mehreren Mundmerkmalen eines Benutzers aufzunehmen.

4. Vorrichtung nach einem der vorstehenden Ansprüche, wobei die Bildaufnahmevorrichtung eine Beleuchtungsvorrichtung (19) umfasst, die dazu konfiguriert ist, in Verwendung einen Teil des Benutzers zu beleuchten, und wobei vorzugsweise die Steuereinheit dazu eingerichtet ist, die Bildaufnahmevorrichtung zu steuern, um einen Zeitpunkt der Beleuchtung mit der Vibrationswellenform zu synchronisieren.

5. Vorrichtung nach einem der vorstehenden Ansprüche, wobei die Steuereinheit (18) dazu eingerichtet ist, eine Bildaufnahmebildrate der Bildaufnahmevorrichtung weiter basierend auf der Vibrationsfrequenz der Körperpflegevorrichtung zu steuern.

6. Vorrichtung nach einem der vorstehenden Ansprüche, wobei die Steuereinheit (18) weiter dazu konfiguriert ist, die Vibrationsmittel zu steuern, um die Vibrationsfrequenz der Körperpflegevorrichtung basierend auf einer Bildaufnahmebildrate der Kamera auszulegen.

7. Vorrichtung nach einem der vorstehenden Ansprüche, wobei die Steuereinheit (18) dazu eingerichtet ist, die Belichtungsparameter über einen Rahmen der Bildaufnahmevorrichtung zu verteilen, vorzugsweise zeilenweise.

8. Vorrichtung nach Anspruch 7, wobei die Steuereinheit (18) weiter dazu eingerichtet ist, den Belichtungsparameter über den Rahmen der Bildaufnahmevorrichtung hinweg zu variieren.

9. Vorrichtung nach einem der vorstehenden Ansprüche, wobei die Körperpflegevorrichtung eine Zahnbürste umfasst.

10. Vorrichtung nach einem der vorstehenden Ansprüche, wobei die Körperpflegevorrichtung ein Mundstück umfasst.

11. Verfahren zum Steuern einer Körperpflegevorrichtung, die Körperpflegevorrichtung umfassend:
Vibrationsmittel, die dazu geeignet sind, die Körperpflegevorrichtung in Vibrationen zu versetzen, sodass die Körperpflegevorrichtung in Verwendung mit einer periodischen Vibrationswellenform vibriert; und eine Bildaufnahmevorrichtung, die dazu eingerichtet ist, in Verwendung Bilder von einem oder mehreren Merkmalen eines Benutzers aufzunehmen, wobei das Verfahren umfasst:
Steuern eines Belichtungsparameters der Bildaufnahmevorrichtung basierend auf einem Parameter der periodischen Vibrationswellenform;
Bestimmen eines Zielbelichtungsdauerwerts auf der Grundlage des Parameters der periodischen Vibrationswellenform und mindestens eines Auflösungsparameters der Bildaufnahmevorrichtung;
Einstellen der Belichtungsdauer für die Bildaufnahme auf den Zielbelichtungsdauerwert, und wobei vorzugsweise der mindestens eine Auflösungsparameter einen oder mehrere der folgenden Parameter umfasst: Sensorpixelbreite, Sensorpixellänge, Sensorabstand und Sensordichte.

12. Computerprogramm, umfassend ein Computerprogrammcodemittel, das dazu eingerichtet ist, wenn das Computerprogramm auf einer Körperpflegevorrichtung nach Anspruch 1 läuft, das Verfahren nach Anspruch 11 zu implementieren.

## Revendications

1. Dispositif de soins personnels comprenant :
des moyens vibratoires (12) adaptés pour faire vibrer le dispositif de soins personnels de sorte que, en cours d'utilisation, le dispositif de soins personnels vibre avec une forme d'onde de vibration périodique ;
un dispositif de capture d'image (16) adapté, en cours d'utilisation, pour capturer des images d'une ou plusieurs caractéristiques d'un utilisateur ; et
une unité de commande (18) adaptée pour commander un paramètre d'exposition du dispositif de capture d'image sur la base d'un paramètre de la forme d'onde de vibration périodique ;
dans lequel le paramètre d'exposition comprend une durée d'exposition de capture d'image du dispositif de capture d'image, et dans lequel l'unité de commande est adaptée pour régler la durée d'exposition de capture d'image pour qu'elle soit inférieure ou égale à une valeur de durée d'exposition cible ; et
dans lequel l'unité de commande (18) est adaptée pour déterminer la valeur de durée d'exposition cible sur la base du paramètre de la forme d'onde de vibration périodique et d'au moins un paramètre de résolution du dispositif de capture d'image, de préférence dans lequel le au moins un paramètre de résolution comprend un ou plusieurs parmi : la largeur des pixels du capteur ; la longueur des pixels du capteur ; l'espacement des capteurs ; et la densité des capteurs.

2. Dispositif selon la revendication 1, dans lequel la valeur de durée d'exposition cible est inférieure ou égale au temps nécessaire à la capture d'image par le dispositif de capture d'image pour se déplacer sur un nombre de pixels prédéterminé, de préférence dans lequel le nombre de pixels prédéterminé est égal à un pixel.

3. Dispositif selon l'une quelconque des revendications précédentes, dans lequel le dispositif de soins personnels comprend un dispositif de soins buccaux, et dans lequel le dispositif de capture d'image (16) est adapté, en cours d'utilisation, pour capturer des images d'une ou plusieurs caractéristiques buccales d'un utilisateur.

4. Dispositif selon l'une quelconque des revendications précédentes, dans lequel le dispositif de capture d'image comprend un dispositif d'éclairage (19) configuré, en cours d'utilisation, pour éclairer une partie de l'utilisateur, et de préférence dans lequel l'unité de commande est adaptée pour commander le dispositif de capture d'image afin de synchroniser une synchronisation d'éclairage avec la forme d'onde de vibration.

5. Dispositif selon l'une quelconque des revendications précédentes, dans lequel l'unité de commande (18) est adaptée pour commander une fréquence de trame de capture d'image du dispositif de capture d'image en outre sur la base de la fréquence de vibration du dispositif de soins personnels.

6. Dispositif selon l'une quelconque des revendications précédentes, dans lequel l'unité de commande (18) est en outre configurée pour commander les moyens vibratoires pour ajuster la fréquence de vibration du dispositif de soins personnels sur la base d'une fréquence de trame de capture d'image de la caméra.

7. Dispositif selon l'une quelconque des revendications précédentes, dans lequel l'unité de commande (18) est adaptée pour distribuer le paramètre d'exposition sur une trame du dispositif de capture d'image, de préférence ligne par ligne.

8. Dispositif selon la revendication 7, dans lequel l'unité de commande (18) est en outre adaptée pour faire varier le paramètre d'exposition sur l'ensemble de la trame du dispositif de capture d'image.

9. Dispositif selon l'une quelconque des revendications précédentes, dans lequel le dispositif de soins personnels comprend une brosse à dents.

10. Dispositif selon l'une quelconque des revendications précédentes, dans lequel le dispositif de soins personnels comprend un embout buccal.

11. Procédé de commande d'un dispositif de soins personnels, le dispositif de soins personnels comprenant :
des moyens vibratoires adaptés pour faire vibrer le dispositif de soins personnels de sorte que, en cours d'utilisation, le dispositif de soins personnels vibre avec une forme d'onde de vibration périodique ; et un dispositif de capture d'image adapté, en cours d'utilisation, pour capturer des images d'une ou plusieurs caractéristiques d'un utilisateur, dans lequel le procédé comprend :
la commande d'un paramètre d'exposition du dispositif de capture d'image sur la base d'un paramètre de la forme d'onde de vibration périodique ;
la détermination d'une valeur de durée d'exposition cible sur la base du paramètre de la forme d'onde de vibration périodique et d'au moins un paramètre de résolution du dispositif de capture d'image ;
le réglage de la durée d'exposition de capture d'image sur la valeur de durée d'exposition cible, et de préférence dans lequel le au moins un paramètre de résolution comprend un ou plusieurs parmi : la largeur des pixels du capteur ; la longueur des pixels du capteur ; l'espacement des capteurs ; et la densité du capteur.

12. Programme informatique comprenant un moyen de code de programme informatique qui est adapté, lorsque ledit programme informatique est exécuté sur le dispositif de soins personnels selon la revendication 1 pour mettre en œuvre le procédé selon la revendication 11.
